# EUROPÄISCHE PATENTANMELDUNG

(11) **EP 1 764 079 A1**
(43) Veröffentlichungstag der Anmeldung: **21.03.2007**
(21) Anmeldenummer: 06120654.6
(22) Anmeldetag: 14.09.2006
(51) Int. Cl.: A61K 8/35, A61K 8/72, A61Q 19/04, A61Q 17/00

(54) **Kosmetische Selbstbräunungsformulierungen**

(30) Priorität: 16.09.2005 DE 102005045141
(71) Anmelder: Beiersdorf AG, 20253 Hamburg (DE)
(72) Erfinder: Kallmayer, Volker, 22393, Hamburg (DE); Lanzendoerfer, Ghita, 90491, Nürnberg (DE); Mannes, Annika, 22459, Hamburg (DE); Mocigemba, Nicole, 22529, Hamburg (DE); Riedel, Heidi, 22529, Hamburg (DE); Schäfer, Jessica, 22299, Hamburg (DE); Viala, Sophie, 20257, Hamburg (DE)

(57) **Zusammenfassung**

Kosmetische Selbstbräunungszubereitung, welche Dihydroxyaceton enthält und dadurch gekennzeichnet sind, daß sie ferner ein oder mehrere Ligninsulfonate enthält.

## Beschreibung

Die vorliegende Erfindung betrifft kosmetische Selbstbräunungszubereitungen mit einem Gehalt an Dihydroxyaceton und einem oder mehreren Ligninsulfonaten.

Die natürliche Bräunung durch Einwirkung der Sonnenstrahlen ist zwar die gefährlichste, aber nach wie vor die häufigste und beliebteste Methode, um eine gewünschte Pigmentierung der Haut zu erzielen. Die Sonne läßt sich praktisch überall genießen und ausnützen: Beim Badeurlaub an der Sonnenküste, beim Skifahren, beim Bergwandern, beim Freizeitsport oder auch nur beim kurzen Sonnen während der Mittagspause. Ein Sonnenbad wirkt - in Maßen genossen - angenehm entspannend, beruhigend und erholsam. Es verbessert die Stimmungslage und Laune. Nicht zu vergessen sind die positiven Auswirkungen auf den gesamten Organismus über die Anregung verschiedener Stoffwechselvorgänge.

Daneben gehen von den Sonnenstrahlen allerdings auch eine Reihe nachteiliger Wirkungen aus: Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut. Dementsprechend sind viele Verbraucher im Umgang mit der Sonne vorsichtiger geworden. Ihre Einstellung zum Sonnenbaden hat sich geändert: Braunsein um jeden Preis ist out - gesunde Bräune ist in.

Unter künstlicher Bräunung sind alle Maßnahmen zu verstehen, die der Haut ohne Einwirkung von Sonnenstrahlen ein gebräuntes Aussehen verleihen. Hierfür sind mehrere Methoden prinzipiell denkbar:
- Als einfachste Methode ist die Anwendung von Schminken anzuführen. Dabei handelt es sich um Farbstoffpräparationen, die lediglich physikalisch an der Hautoberfläche haften, im Gegensatz zu den selbstbräunenden Präparaten, die eine echte chemische Bindung mit Bestandteilen der Hornschicht eingehen.
- Eine weitere Methode der künstlichen Bräunung ist die Einnahme von Carotinoiden in Form von Kapseln oder Dragees, welche zu einer gelblich-braunen Hautfärbung führt.
- Eine dritte Methode der künstlichen Bräunung ist die Bestrahlung der Haut unter Verwendung technisch hergestellter Lichtquellen, die in der Lage sind, ultraviolette Strahlen unterschiedlicher Art und Stärke zu emittieren.

Während die Bräunung der Haut mit ultravioletten Strahlen, wie sie im natürlichen Sonnenspektrum vorkommen oder von künstlichen Lichtquellen erzeugt werden, bei zu langer Einwirkung und Überschreitung einer bestimmter Dosis zu nicht unerheblichen Hautschäden führen kann, ist die Anwendung von Schminken, Selbstbräunungscremes und Beta-Carotenhaltigen Bräunungspillen als harmlos und unbedenklich einzustufen. Wer also lichtbedingte Hautschäden, wie Sonnenbrand, chronische Lichtschäden oder mögliches Karzinomrisiko vermeiden will, auf die modisch erwünschte Bräune jedoch nicht verzichten möchte, der muß auf solche Methoden der Bräunung zurückgreifen, die ohne die Anwendung künstlich erzeugter oder natürlicher ultravioletter Strahlen auskommen. Gleiches gilt für Personen, die sich - aufgrund krankhafter Hautreaktionen dem Ultraviolett nicht aussetzen dürfen.

Daß manche Substanzen mit dem Keratin von (Tier-) Haut, Haaren und Wolle eine chemische Farbreaktion eingehen, wußte man schon aus der Textilchemie und der Küppen-Färberei. Letztlich hat sich hier für die kosmetische Anwendung eine Klasse von Wirkstoffen durchgesetzt, die den Anforderungen an moderne kosmetische Mittel gerecht wird. Diese Wirkstoffe sind nicht reizend, leicht zu handhaben, und der resultierende Farbton ist lichtecht und nicht abwaschbar.
Verwendet werden Substanzen mit einer benachbarten Keto-Alkohol- oder Aldehyd-Alkoholgruppierung, also Ketole oder Aldole die überwiegend zur Klasse der Zucker gehören. Der wichtigste und nach wie vor am häufigsten eingesetzt Grundstoff ist das Dihydroxyaceton (DHA), ein im menschlichen Körper vorkommender dreiwertiger Zucker. DHA reagiert mit den Proteinen und Aminosäuren der Hornschicht, wobei sich die Aminogruppe der Aminosäuren mit der Ketogruppe des DHA im Sinne einer Maillard-Reaktion umsetzt (Schiffsche Base). Der Farbton kommt über eine Polymerisation zustande, deren einzelne Reaktionsschritte noch nicht ganz abgeklärt sind.

Diese Art der Bräunung ist eine rein chemische Reaktion der Hornschicht und hat mit einer Neubildung von Melanin nichts zu tun. Die mit DHA am Keratin entstehenden gefärbten Produkte werden als Melanoide bezeichnet. Versuche haben gezeigt, daß die Bräunungseigenschaften von DHA stark durch die Formulierung beeinflußt werden können: So erhöht z. B. ein hoher Wasseranteil die Bräunungsintensität; Unterschiede im Farbton ergeben sich in Abhängigkeit vom pH-Wert; Liposome als Träger wirken als Bräunungsverstärker.

Die Intensität der Verfärbung ist auch von der Dicke der Hornschicht abhängig. Stark verhornte Stellen wie Handballen und Fußsohlen werden stärker angefärbt. Das Gesicht nimmt weniger Farbton an als die Extremitäten, allerdings sind die individuellen Unterschiede sehr groß. Bei 10 bis 15 Prozent aller Menschen wird die Haut infolge einer besonderen Hautkonstitution mit DHA überhaupt nicht angefärbt.

Wie an histologischen Schnitten gezeigt werden konnte, nehmen nur die obersten Schichten der Hornschicht den Farbton an. Die erste Bräunung tritt nach etwa zwei bis vier Stunden ein, nach ca. sechs Stunden ist der Bräunungsvorgang abgeschlossen, wobei eine Anwendungswiederholung nach zwei bis drei Stunden zu optimalen Ergebnissen führt. Die Verfärbung hält - je nach der Geschwindigkeit der Abstoßung der oberen Hornschichten - drei bis sieben Tage, nimmt dann langsam ab, um binnen fünf bis fünfzehn Tagen völlig zu verschwinden.

Selbstbräunende Präparate mit Dihydroxyaceton oder chemisch ähnlich reagierenden Substanzen sind absolut unschädlich. Es wird nur die tote Zellschicht der obersten Hornlagen der Haut angefärbt, eine Schädigung tieferer Hautschichten ist ausgeschlossen. Da Nebenwirkungen nicht auftreten - die Substanzen sind nicht toxisch und Sensibilisierungen äußerst selten - ist gegen eine langdauernde Anwendung selbstbräunender Zubereitungen überhaupt nichts einzuwenden.

Ein entscheidender Nachteil DHA-haltiger Selbstbräunungszubereitungen ist allerdings, daß es dem Verwender zum Zeitpunkt des Auftrags nicht möglich ist, die Gleichmäßigkeit des Auftrags zu kontrollieren. Erst bei Erreichen eines deutlichen Bräunungsgrads wird offenbar, ob das Produkt gleichmäßig appliziert wurde. An dieser Stelle kann jedoch nicht mehr korrigierend eingegriffen werden, und der Verwender muss so lange mit der ungleichmäßigen Bräune leben, bis im Rahmen der natürlichen Hautabschilferung nach ca. 2 Wochen die DHA induzierte Bräune wieder vollständig verblasst ist. Diesem Missstand könnte man beispielsweise dadurch abhelfen, dass man die Zubereitungen einfärbt, z. B. durch den Einsatz von Eisenoxiden, welche auch in Dekorativen Kosmetik-Produkten (z. B. Foundations) verwendet werden. Eisenoxide beschleunigen jedoch den Abbau des DHAs und führen so zu einer Verschiebung des DHA-induzierten Farbtons ins Gelbliche sowie zu einem schnelleren Verderb des Produktes selbst, was sich besonders negativ an der damit einhergehenden Verfärbung und Geruchsveränderung für den Verbraucher bemerkbar macht.

Ein weiterer Nachteil DHA-haltige Selbstbräunungsuzubereitungen des Standes der Technik ist, dass diese zu einer Austrockung der oberen Hautschicht führen, was ebenfalls zu ungleichmäßiger Bräune sowie einem unangenehmen Spannungsgefühl beim Verwender führen kann.

Aufgabe der vorliegenden Erfindung war es daher, auf einfache und preiswerte Weise zu Selbstbräunungszubereitungen (insbesondere zu O/W-Formulierungen) zu gelangen, welche die genannten Nachteile nicht aufweisen.

Überraschend und für den Fachmann nicht vorauszusehen war, daß eine
kosmetische Selbstbräunungszubereitung, welche Dihydroxyaceton enthält und dadurch
gekennzeichnet sind, daß sie ferner ein oder mehrere Ligninsulfonate enthält,
den Nachteilen des Standes der Technik abhelfen würde.

Die Zubereitungen gemäß der vorliegenden Erfindung stellen in jeglicher Hinsicht überaus befriedigende Präparate dar, welche nicht auf eine eingeschränkte Rohstoffauswahl begrenzt sind. Sie zeigen sehr gute sensorische und kosmetische Eigenschaften, wie beispielsweise die Verteilbarkeit auf der Haut oder das Einzugsvermögen in die Haut bei gleichzeitig hervorragenden Hautpflegedaten. Die Zubereitungen gemäß der vorliegenden Erfindung zeichen sich ferner insbesondere dadurch aus, daß sie einen angenehm weichen Film auf der Haut bilden, der einer Austrocknung entgegenwirkt.

Ligninsulfonsäure ist das beim Sulfit-Aufschluß von Holz zur Gewinnung von Cellulose anfallende Reaktionsprodukt aus nativem Lignin und schwefliger Säure. Bei diesem Aufschlußverfahren wird Lignin - ein hochmolekularer, aromatischer Stoff, der in verholzenden Pflanzen die Räume zwischen den Zellmembranen ausfüllt und diese hierdurch versteift und zu Holz werden läßt - an den C₃-Seitenketten der Phenylpropan-Grundeinheiten sulfoniert.

In Abhängigkeit von den beim Aufschlußverfahren verwendeten Basen resultieren wasserlösliche Natrium-, Ammonium-, Calcium- oder Magnesium-Salze der Ligninsulfonsäure. Angaben zum M_{R} der Ligninsulfonsäure. variieren mit Werten von ca. 10.000 bis 200.000; die Anzahl der Sulfonsäure-Gruppen beträgt ca. 2 pro 5 bis 8 Phenylpropan-Einheiten. Ligninsulfonsäure und ihre Salze, die Ligninsulfonate, sind Hauptbestandteil der SulfitAblaugen, aus denen sie als braune Pulver isoliert werden können. Die isolierten Produkte finden breite technische Verwendung, z. B. als Dispergatoren in Zement- und Gipsmörteln, als Bohrspülflüssigkeiten sowie als Flotationshilfsmittel, als Zusatz bei der Futtermittelpalettierung sowie zur Formsandbindung in Gießereien.

Vorteilhafte Ligninsulfonate im Sinne der vorliegenden Erfindung sind Calciumligninsulfonat (CAS-Nr. :8061-52-7) und Natriumligninsulfonat (CAS-Nr.: 8061-51-6), insbesondere die unter den Handelbezeichnungen Ultrazine NA und Vanisperse CB bei der Firma Borregaard Lignotech erhältlichen Ligninsulfonate.

Besonders vorteilhafte Zubereitungen im Sinne der vorliegenden Erfindung enthalten 0,01 bis 20 Gew.-%, vorteilhaft 0,5 bis 15 Gew.-%, besonders 1 bis 7 Gew.-% an einem oder mehreren Ligninsulfonaten (jeweils bezogen auf das Gesamtgewicht der Zubereitung).

Es ist vorteilhaft im Sinne der vorliegenden Erfindung, den Gehalt an Dihydroxyaceton wie 0,01 bis 20, vorteilhaft 0,5 bis 15, besonders von 1 bis 10 Gew.-% zu wählen (jeweils bezogen auf das Gesamtgewicht der Zubereitung).

Es ist bevorzugt, wenn die erfindungsgemäßen Zubereitungen O/W-Emulsionen darstellen, insbesondere O/W-Emulsionen, welche eine Tröpfchengröße der inneren Phase von mehr als 500 nm, besonders bevorzugt von mehr als 1000 nm aufweisen.

Erfindungsgemäße O/W-Emulsionen enthalten bevorzugt einen oder mehrere Emulgatoren, welche zur Herstellung derartiger Zubereitungen geeignet sind.

Bevorzugt werden der oder die Emulgatoren aus der Gruppe der polyethoxylierten Fettsäureester mit einer Kettenlänge von 10 bis 30 Kohlenstoffatomen und mit einem Ethoxylierungsgrad von 5 bis 100 gewählt. Besonders vorteilhaft sind beispielsweise polyethoxylierte Stearinsäureester sowie polyethoxyliertes Ricinusöl (engl. castoroil).

Ferner vorteilhaft werden der oder die Emulgatoren aus der Gruppe der Mischester gesättigter, unverzweigter Fettsäuren mit monomethoxylierter Glucose und (ggf. partial veresterten) Polyglycerinen gewählt (z. B. Polyglyceryl-3 Methylglucose Distearat). Vorteilhafte Emulgatoren zur Herstellung von O/W-Emulsionen im Sinne der vorliegenden Erfindung sind ferner Natrium Cetearylsulfat sowie Glycerylstearat, Glycerylisostearat, Glyceryldiisostearat, Glyceryloleat, Glycerylpalmitat, Glycerylmyristat, Glyceryllanolat und/oder Glyceryllaurat.

Es kann ferner vorteilhaft im Sinne der vorliegenden Erfindung sein, zur Herstellung einer erfindungsgemäßen O/W-Emulsion zusätzlich zu dem oder den Emulgator(en) einen oder mehrere Coemulgatoren aus der Gruppe der Fettalkohole mit einer Kettenlänge von 10 bis 40 Kohlenstoffatomen zu verwenden. Besonders bevorzugter Coemulgator ist Cetearylalkohol.

Besonders vorteilhaft im Sinne der vorliegenden Erfindung sind ferner selbstschäumende, schaumförmige, nachschäumende oder schäumbare kosmetische Zubereitungen.

Unter "selbstschäumend", "schaumförmig", "nachschäumend" bzw. "schäumbar" sind Zubereitungen zu verstehen, aus welchen Schäume - sei es bereits während des Herstellprozesses, sei es bei der Anwendung durch den Verbraucher oder auf andere Weise - durch Eintrag eines oder mehrerer Gase im Prinzip herstellbar sind. In derartigen Schäumen liegen die Gasbläschen (beliebig) verteilt in einer (oder mehreren) flüssigen Phase(n) vor, wobei die (aufgeschäumten) Zubereitungen makroskopisch nicht notwendigerweise das Aussehen eines Schaumes haben müssen. Erfindungsgemäße (aufgeschäumte) kosmetische Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße Schäume - insbesondere dann, wenn die Gasbläschen zu klein sind, um unter einem Lichtmikroskop erkannt zu werden - auch an der starken Volumenzunahme des Systems erkennbar.

Deratige Zubereitungen enthalten im Sinne der vorliegenden Erfindung vorteilhaft ein Emulgatorsystem, welches aus
A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen. Ferner ist es erfindungsgemäß besonders bevorzugt, wenn derartige Schaumformulierungen frei sind von Mono- oder Diglycerylfettsäureestern. Insbesondere bevorzugt sind Schaumformulierungen, welche kein Glycerylstearat, Glycerylisostearat, Glyceryldüsostearat, Glyceryloleat, Glycerylpalmitat, Glycerylmyristat, Glyceryllanolat und/oder Glyceryllaurat enthalten, da diese Verbindungen die Schaumqualität negativ beeinflussen bzw. den Schaum zerstören.

Besonders vorteilhaft im Sinne der vorliegenden Erfindung sind ferner sprühbare O/W-Emulsionen, insbesondere O/W-Mikroemulsionen.

Die Tröpfchendurchmesser der gewöhnlichen "einfachen", also nichtmultiplen Emulsionen liegen im Bereich von ca. 1 µm bis ca. 50 µm. Solche "Makroemulsionen" sind, ohne weitere färbende Zusätze, milchigweiß gefärbt und opak. Feinere "Makroemulsionen", deren Tröpfchendurchmesser im Bereich von ca. 0,5 µm bis ca. 1 µm liegen, sind, wiederum ohne färbende Zusätze, bläulichweiß gefärbt und opak. Solche "Makroemulsionen" haben für gewöhnlich ein hohe Viskosität.

Der Tröpfchendurchmesser von Mikroemulsionen im Sinne der vorliegenden Erfindung dagegen liegt im Bereich von etwa 50 bis etwa 500 nm. Derartige Mikroemulsionen sind bläulichweiß gefärbt bis transluzent und meist niedrigviskos. Die Viskosität vieler Mikroemulsionen vom O/W-Typ ist vergleichbar mit der des Wassers.

Vorteil von Mikroemulsionen ist, dass in der dispersen Phase Wirkstoffe wesentlich feiner dispers vorliegen können als in der dispersen Phase von "Makroemulsionen". Ein weiterer Vorteil ist, dass sie aufgrund ihrer niedrigen Viskosität versprühbar sind. Werden Mikroemulsionen als Kosmetika verwendet, zeichnen sich entsprechende Produkte durch hohe kosmetische Eleganz aus.

Erfindungsgemäß vorteilhaft sind insbesondere O/W-Mikroemulsionen, welche mit Hilfe der sogenannten Phaseninversionstemperatur-Technologie erhältlich sind und mindestens einen Emulgator (Emulgator A) enthalten, welcher gewählt wird aus der Gruppe der Emulgatoren mit folgenden Eigenschaften:
- ihre Lipophilie ist abhängig von der Temperatur, dergestalt dass durch Erhöhung der Temperatur die Lipophilie zunimmt und durch Senkung der Temperatur die Lipophilie des Emulgators abnimmt.

Vorteilhafte Emulgatoren A sind z. B. polyethoxilierte Fettsäuren (PEG-100 Stearat, PEG-20 Stearat, PEG-150 Laurath, PEG-8 Distearat und dergleichen) und/oder polyethoxilierte Fettalkohole (Cetearath-12, Cetearath-20, Isoceteth-20, Beheneth-20, Laureth-9 etc.) und/oder Alkylpolyglycoside (Cetearyl Glycoside, Stearyl Glycoside, Palmityl Glycoside etc.).

Sofern die Phaseninversion im wesentlichen durch Variation der Temperatur eingeleitet wird, sind O/W-Emulsionen, insbesondere O/W-Mikroemulsionen erhältlich, wobei die Größe der Öltröpfchen im wesentlichen durch die Konzentration des oder der eingesetzten Emulgatoren bestimmt wird, dergestalt, dass eine höhere Emulgatorkonzentration kleinere Tröpfchen bewirkt und geringere Emulgatorkonzentration zu größeren Tröpfchen führt. Die Tröpfchengrößen liegen in der Regel zwischen 20 und 500 nm.

Es ist im Sinn der vorliegenden Erfindung gegebenenfalls vorteilhaft, weitere, nicht unter die Definition des Emulgators A fallende, W/O und/oder O/W-Emulgatoren zu verwenden, beispielsweise um die Wasserfestigkeit der Zubereitungen gemäß der vorliegenden Erfindung zu erhöhen. Hier können z. B. Alkylmethiconcopolyole und/oder Alkyl-Dimethiconcopolyole (insbesondere Cetyl Dimethicone Copolyol, Lauryl Methicone Copolyol), W/O-Emulgatoren (wie z. B. Sorbitanstearat, Glycerylstearat, Glycerolstearat, Sorbitanoleat, Lecithin, Glycerylisostearat, Polyglyceryl-3-Oleat, Polyglyceryl-3 Diisostearat, PEG-7-hydriertes Ricinusöl, Polyglyceryl-4-isostearat, Acrylat/ C₁₀₋₃₀-Alkylacrylat-Crosspolymer, Sorbitanisostearat, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Glycol Distearat, Polyglyceryl-3-dipolyhydroxystearat) und/oder Fettsäureester der Schwefel-oder Phosphorsäure (Cetylphosphat, Trilaureth-4 Phosphat, Trioleth-8-Phosphat, Stearylphosphat, Cetearylsulfat etc.) verwendet werden.

Weitere vorteilhafte sprühbare O/W-Emulsionen im Sinne der vorliegenden Erfindung sind dünnflüssige kosmetische Hydrodispersionen, welche mindestens eine Ölphase und mindestens eine Wasserphase enthalten, wobei die Zubereitung durch mindestens einen Gelbildner stabilisiert wird und nicht notwendigerweise Emulgatoren enthalten muss, aber einen oder mehrere Emulgatoren enthalten kann.

Vorteilhafte Gelbildner für derartige Zubereitungen sind beispielsweise Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die Pemulen® Typen TR 1, TR 2 und TRZ von der Fa. Goodrich (Noveon).

Auch Carbopole sind vorteilhafte Gelbildner für derartige Zubereitungen. Carbopole sind Polymere der Acrylsäure, insbesondere auch Acrylat-Alkylacrylat-Copolymere. Vorteilhafte Carbopole sind beispielsweise die Typen 907, 910, 934, 940, 941, 951, 954, 980, 981, 1342, 1382, 2984 und 5984. ebenso die ETD-Typen 2020, 2050 und Carbopol Ultrez 10. Weitere vorteilhafte Gelbildner für derartige Zubereitungen sind Xanthan Gummi, Cellulose Derivate und Johannisbrotkernmehl.

Als mögliche (fakultative) Emulgatoren können ethoxilierte Fettalkohole oder ethoxilierte Fettsäuren (insbesondere PEG-100 Stearat, Ceteareth-20) und/oder andere nichtionische oberflächenaktive Substanzen verwendet werden.

Ferner vorteilhaft können die sehr dünnflüssigen bis sprühbaren Emulsionen auch W/O- bzw. Wasser-in-Silikonöl- (W/S-) Emulsionen sein. Insbesondere vorteilhaft sind W/O- bzw. W/S-Emulsionen, die
- mindestens einen Silikonemulgator (W/S) mit einem HLB-Wert ≤ 8 und/oder mindestens einen W/O-Emulgator mit einem HLB-Wert < 7 und
- mindestens einen O/W-Emulgator mit einem HLB-Wert > 10 enthalten.
Derartige Zubereitungen enthalten ferner mindestens 20 Gew.-% Lipide, wobei die Lipidphase vorteilhaft auch Silikonöle enthalten bzw. sogar ganz aus solchen bestehen kann.

Der oder die Silikonemulgatoren kann vorteilhaft aus der Gruppe der Alkylmethiconcopolyole und/oder Alkyl-Dimethiconcopolyole gewählt werden (z. B. Dimethiconcopolyole, welche von der Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden, Cetyl Dimethiconcopolyol [Goldschmidt AG / ABIL® EM 90], Cyclomethicon Dimethiconcopolyol [Goldschmidt AG / ABIL® EM 97], Laurylmethiconcopolyol [Dow Corning Ltd. / Dow Corning® 5200 Formulation Aid], Octyl Dimethicon Ethoxy Glucosid [Firma Wacker].

Der oder die W/O-Emulgatoren mit einem HLB-Wert < 7 kann vorteilhaft aus folgender Gruppe gewählt werden: Sorbitanstearat, Sorbitanoleat, Lecithin, Glyceryllanolat, Lanolin, hydriertem Ricinusöl, Glycerylisostearat, Polyglyceryl-3-Oleat, Pentaerythrithylisostearat, Methylglucosedioleat, Methylglucosedioleat im Gemisch mit Hydroxystearat und Bienenwachs, PEG-7-hydriertes Ricinusöl, Polyglyceryl-4- isostearat, Hexyllaurat, Acrylat/ C₁₀₋₃₀-Alkylacrylat-Crosspolymer, Sorbitanisostearat, Polyglyceryl-2-dipolyhydroxystearat, Polyglyceryl-3-Diisostearat, PEG-30-dipolyhydroxystearat, Diisostearoylpolyglyceryl-3-diisostearat, Polyglyceryl-3-dipolyhydroxystearat, Polyglyceryl-4-dipolyhydroxystearat, Polyglyceryl-3-dioleat.

Der oder die O/W-Emulgatoren mit einem HLB-Wert > 10 kann vorteilhaft aus folgender Gruppe gewählt werden: Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65, Polysorbate-100, Glycerylstearat im Gemisch mit PEG-100 Stearat, Ceteareth-3, Isostearylglycerylether, Cetylstearylalkohol im Gemisch mit Natrium Cetylstearylsulfat, PEG-40-Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10, PEG-20-Stearat, Steareth-21, Steareth-20, Isosteareth-20, PEG-45/ Dodecylglycol-Copolymer, Methoxy-PEG-22/Dodecylglycol-Copolymer, Glycerylstearat SE, Ceteth-20, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Isosteareth-10, Polyglyceryl-2-sesquiisostearat, Ceteth-10, Isoceteth-20, Glycerylstearat im Gemisch mit Ceteareth-20, Ceteareth-12, Cetylstearylalkohol und Cetylpalmitat, PEG-30-Stearat, PEG-40-Stearat, PEG-100-Stearat.

Vorteilhaft im Sinne der Erfindung kann es auch sein, die Wasser-in-Silikonöl- (W/S-) Emulsionen zu verdicken. Neben den üblicherweise in der Kosmetik eingesetzen und dem Fachmann bekannten Verdickern sind ferner vor allem Siloxanelastomere hierzu geeignet. Erfindungsgemäß bevorzugt sind dementsprechend auch W/S- Emulsionen, welche ein oder mehrere Siloxanelastomere enthalten.

Siloxalelastomere sind feste, elastomere Polyorganosiloxane oder Organopolysiloxane die teilweise oder vollständig vernetzt vorliegen und zumeist eine dreidimensionale Struktur aufweisen.

Erfindungsgemäß vorteilhafte Ausführungsformen der vorliegenden Erfindung sind dadurch gekennzeichnet, dass feste elastomere Polyorganosiloxane oder Organopolysiloxane, im folgenden als Siloxanelastomere bezeichnet, zum Einsatz kommen. Siloxalelastomere sind teilweise oder vollständig vernetzt und weisen zumeist eine dreidimensionale Struktur auf. Sie sind erhältlich durch eine Reaktion von vinyl-endständigem Polymethylsiloxan und Methylhydrodimenthylsiloxan 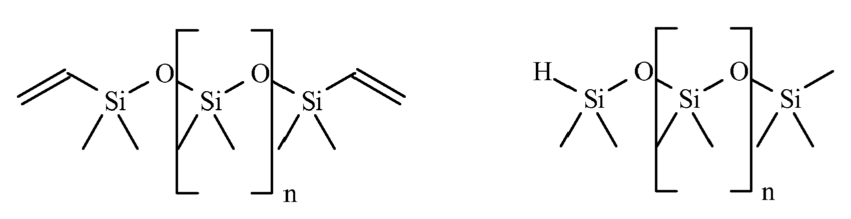 oder auch durch Reaktion von Hydroxy-endständigem Dimethylpolysiloxan und Trimethylsiloxyendständigem Methylpolysiloxan: 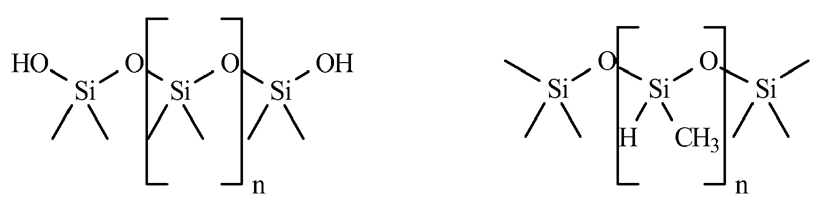 oder durch Reaktion eines α, ω- Dienes mit der Formel CH₂ =CH(CH₂)ₓCH=CH₂, mit x =1 - 20 und Methylhydrodimethylsiloxan ("New Developments in Silicone Elastomers for Skin Care", Michael Starch, Dow Corning, 2002)

Erfindungsgemäß bevorzugt wird als Elastomer DC 9040 der Firma Dow Corning eingesetzt.

Erfindungsgemäß bevorzugt weist das erfindungsgemäße Siloxanelastomer eine Vernetzung durch α, ω-Diene mit der Formel CH₂ =CH(CH₂)ₓCH=CH₂, mit x =1 - 20 auf.

Ferner vorteilhaft im Sinne der vorliegenden Erfindung sind wäßrig-alkoholische Lösungen. Sie können von 0 Gew.-% bis 90 Gew.-% Ethanol enthalten. Wäßrig-alkoholische Lösungen können im Sinne der vorliegenden Erfindung vorteilhaft auch Lösungsvermittler enthalten, wie z. B. PEG-40 oder PEG-60 hydrogeniertes Ricinusöl.

Alle beschriebenen Zubereitungen sind mit den verschiedensten Ölen erhältlich. Wie dem Fachmann bekannt, sind jedoch spezifische Öle, in Abhängigkeit der jeweiligen Produktform, bevorzugt einzusetzen sind. Auch ist eine Mischungen der Öle ist denkbar und für verschiedene gewünschte Eigenschaften der Zubereitung unumgänglich. Auch hier sind dem Fachmann die bevorzugten Mischungen und Konzentrationsbereiche der einzelnen Öle bekannt.

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, PhenylGruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 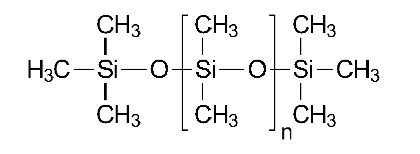 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Besonders vorteilhafte Zubereitungen im Sinne der vorliegenden Erfindung sind O/W-Emulsionen, welche Cyclomethicone enthalten.

Die erfindungsgemäßen kosmetischen Zubereitungen können wie üblich zusammengesetzt sein und zur Behandlung und/oder Pflege der Haut und als Schminkprodukt in der dekorativen Kosmetik dienen.

Zur Anwendung werden die erfindungsgemäßen Zubereitungen in der für Kosmetika üblichen Weise auf die Haut in ausreichender Menge aufgebracht.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Pigmente, die eine färbende Wirkung haben, Verdickungsmittel, anfeuchtende und/oder feuchhaltende Substanzen, Füllstoffe, die das Haut-gefühl verbessern, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, Trisodium NTA, das unter dem Handelsnamen Trilon AS von der Fa. Bayern vertrieben wird, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist. haben

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2020, ETD 2050, Pemulen TR-1 oder -2, Ultrez 10, jeweils einzeln oder in Kombination.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Es ist erfindungsgemäß bevorzugt, den Gehalt an UV-Filtersubstanzen (eine oder mehrere Verbindungen) kleiner als 5 Gew.-%, insbesondere kleiner als 2 Gew.-% zu wählen, jeweils bezogen auf das Gesamtgewicht der Zubereitung. Ganz besonders vorteilhaft sind Zubereitungen im Sinne der vorliegenden Erfindung, welche frei von UV-Filtersubstanzen sind.

Für den Fall, daß ein Gehalt an UV-Filtersubstanzen gewünscht ist, werden diese vorteilhaft aus einer oder mehreren der folgenden Gruppen gewählt: UV-A-, UV-B- und/oder Breitbandfiltersubstanzen sowie organische und/oder anorganische Pigmente als UV-Filtersubstanzen.

Besonders vorteilhafte UV-Filtersubstanzen im Sinne der vorliegenden Erfindung sind:
- Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Symrise erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Symrise erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato- verbindungen, insbesondere das entsprechende Natrium-, Kalium- oder Triethanolammonium-Salz), das auch als Benzol-1,4-di(2-oxo-3-bornylidenmethyl-1 0-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.
- Benzoxazol-Derivate, wie z. B. das 2,4-bis-[5-1 (dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist.
- Hydroxybenzophenone, z. B. der 2-(4'-Diethylamino-2'-hydoxybenzoyl)-benzoesäurehexylester (auch: Aminobenzophenon), welcher unter der Handelsbezeichnung Uvinul A Plus bei der Fa. BASF erhältlich ist.
- Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist; Dioctylbutylamidotriazon (INCI: Diethylhexyl Butamido Triazone), welches unter der Handelsbezeichnung UVASORB HEB bei Sigma 3V erhältlich ist; 4,4',4"-(1,3,5-Triazin-2,4,6-triyitriimino)-tris-benzoesäure-tris(2-ethylhexyiester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird; 2-[4,6-Bis(2,4-dimethylphenyl)-1,3,5-triazin-2-yl]-5-(octyloxy)phenol (CAS Nr.: 2725-22-6).
- Benzotriazole, wie z. B. 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. unter der Handelsbezeichnung Tinosorb® M bei der CIBA-Chemikalien GmbH erhältlich ist.
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Benzalmalonsäure, vorzugsweise 4-Methoxybenzalmalonsäuredi(2-ethylhexyl)-ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter
- Ethylhexyl-2-cyano-3,3-diphenylacrylat (Octocrylen), welches von BASF unter der Bezeichnung Uvinul® N 539 T erhältlich ist.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

| | **1** | **2** | **3** | **4** | **5** | **6** |
|---|---|---|---|---|---|---|
| | Lotion | Creme | Lotion | Schaum | Creme | Lotion |
| Dihydroxyaceton | 3 | 5 | 4 | 2 | 3 | 5 |
| Glycerin | 6 | 10 | 8 | 9 | 10 | 12 |
| Polyglyceryl-3 Methylglucose Distearat | 4 | 4,5 | | | | 3,5 |
| Ligninsulfonat | 5 | 1,5 | 0,5 | 2,5 | 3 | 2 |
| Natrium Cetearylsulfat | | | 1 | | | |
| PEG-40 Stearat | 0,5 | 1 | 0,2 | 2,5 | 1 | 0,1 |
| PEG-40 Ricinusöl | | | 1 | | | |
| Cetearylalkohol | | | 2 | | | |
| Cetylalkohol | 2 | 3 | | 2,5 | | 1,5 |
| Stearylalkohol | | 1 | | | 2 | |
| Sorbitanstearat | | | | 1 | | |
| Glyceryl Stearat | | 2 | | | | |
| Glyceryl Stearat SE | | | 2 | | | |
| Glyceryl Stearat Citrat | | | | | 3 | 1 |
| Myristylmyristat | 2 | 3 | | | 1 | |
| Myristyllactat | | 2 | | | 2 | |
| Stearinsäure | | | | 2,5 | | |
| Alkohol | | | 4 | | 4 | 3 |
| Silikonöl | 5 | | 4 | | 2 | 4 |
| C12-15 Alkylbenzoat | 2 | 1 | 1,5 | | 2 | 0,5 |
| Capryl/Caprintriglycerid | 2 | 3 | | | | |
| Cetylricinoleat | | | | 1,5 | | 1 |
| Octyldodecanol | 3 | 4 | 2 | | 2 | |
| Mineralöl | | 2 | 5 | | | 1 |
| Macadamianussöl | 2 | 1 | 3 | | 3 | 1 |
| Dicapryly Carbonat | 2 | 1,5 | 1 | 1 | | 2 |
| Sorbitol | | 2 | | | | 2 |
| Butylenglykol | | | 2 | | | |
| EDTA | 1 | 0,5 | 1 | 1,5 | 2 | 0,5 |
| Xanthan Gummi | 0,5 | 0,2 | 0,3 | | | 1 |
| C10-30 Alkyl Acrylat Cross-polymer | | | | | | 0,3 |
| Natriumcitrat | | | 0,4 | | 0,3 | |
| Citronensäure | 0,15 | 0,15 | 0,3 | | 0,2 | 0,3 |
| Vitamin E-Acetat | 1 | 0,5 | 0,5 | | 1 | 1 |
| Phenoxyethanol | 0,4 | | 0,4 | 0,5 | | 0,4 |
| Methylparaben | 0,3 | | | 0,3 | 0,2 | |
| Ethylparaben | 0,1 | | | 0,1 | | |
| DMDM Hydantoin | | | 0,1 | | | |
| lodopropynyl Butylcarbamat | | 0,15 | | 0,1 | 0,2 | 0,2 |
| Parfüm | 0,4 | | 0,3 | 0,2 | 0,3 | |
| Wasser | ad 100 | ad 100 | ad 100 | ad 100 | ad 100 | ad 100 |

Der Schaum kann sowohl als Pumpfoamer in einem geeigneten Spendersystem, als auch mit Propan/Isobutan oder Propan/Butan (je 3 : 7) in einem Abfüllverhältnis Konzentrat : Treibgas 97 : 3 bis 80 : 10 gemischt in Aerosolbehältern abgefüllt werden.

Die Lotions können als solche oder auch zur Verwendung auf Tüchern genutzt werden.

| | **7** | **8** | **9** | **10** |
|---|---|---|---|---|
| | Creme | Lotion | Lotion | Lotion |
| Dihydroxyaceton | 5 | 3 | 4 | 6 |
| Glycerin | 10 | 8 | 6 | 12 |
| Ligninsulfonat | 8 | 2,5 | 1,5 | 3 |
| Ceteylalkohol | 1 | 0,5 | | |
| Natrium-Stärke Octenylsuccinat | 1,5 | | 0,5 | 1 |
| Distärkephosphat | 3 | | 1 | 2,5 |
| Silica | | 3 | | 1 |
| PPG-20 Methyl Glucose Ether Distearat | | | 1 | |
| Polysorbat-20 | | | 3 | |
| Methylglucose Sesquistearat | | 2 | | |
| PEG-100 Stearate | 0,5 | 1 | 0,5 | 2 |
| Dimethicon Copolyol | | | | 1,5 |
| Glyceryl Stearat | 2 | | | |
| Alkohol | | | 3 | |
| Cyclomethicone | 2 | 4 | 1 | 3 |
| C12-15 Alkylbenzoat | 5 | | | |
| Capryl/Caprintriglycerid | | | 3 | |
| Octyldodecanol | | 3 | | 2 |
| Mineralöl | | | | 2 |
| Macadamianussöl | 1 | | 2 | |
| Dicapryly Carbonat | | 2 | | |
| Butylenglykol | 3 | | | |
| EDTA | 1 | 1 | 1,5 | 2 |
| Xanthan Gummi | 0,2 | 0,3 | | |
| Natriumcitrat | | 0,4 | | 0,3 |
| Citronensäure | 0,15 | 0,3 | | 0,2 |
| Milchsäure | | | 0,3 | |
| Vitamin E-Acetat | 0,5 | 0,5 | | 1 |
| Phenoxyethanol | 0,1 | 0,4 | 0,5 | |
| Methylparaben | 0,3 | | 0,3 | 0,2 |
| Ethylparaben | | | 0,1 | |
| DMDM Hydantoin | | 0,1 | | |
| lodopropynyl Butylcarbamat | 0,15 | | 0,1 | 0,2 |
| Parfüm | | 0,3 | 0,2 | 0,3 |
| Wasser | ad 100 | ad 100 | ad 100 | ad 100 |

| | **11** | **12** | **13** | **14** |
|---|---|---|---|---|
| | Mikro-emulsion | Mikro-emulsion | Lotion | Mikro-emulsion |
| Dihydroxyaceton | 5 | 3 | 4 | 6 |
| Glycerin | 5 | 8 | 10 | 12 |
| Ligninsulfonat | 8 | 7,5 | 1,5 | 3 |
| Ceteylalkohol | | | 1 | |
| Natrium-Stärke Octenylsuccinat | | | 0,5 | |
| Distärkephosphat | | | 1 | |
| Talk | | | 2,5 | |
| PPG-20 Methyl Glucose Ether Distearat | | | 1 | |
| Polysorbat-20 | | | 3 | |
| Isoceteth-20 | 5 | 5,5 | | 7 |
| PEG-100 Stearate | | | 0,5 | |
| Glyceryl Isostearat | 2,5 | 1,5 | | 3 |
| Alkohol | | | 3 | |
| Macadamianussöl | 0,5 | 1 | | 0,1 |
| Cyclomethicone | 2 | | 1 | 3 |
| C12-15 Alkylbenzoat | 5 | | | |
| Mineralöl | 10 | 8,5 | | 5 |
| Butylenglykol | 3 | | | |
| EDTA | 1 | 1 | 1,5 | 2 |
| Xanthan Gummi | | | 0,15 | |
| Natriumcitrat | 0,1 | 0,4 | | 0,3 |
| Citronensäure | 0,15 | 0,3 | | 0,2 |
| Milchsäure | | | 0,3 | |
| Vitamin E-Acetat | 0,5 | 0,5 | | 1 |
| Phenoxyethanol | 0,1 | 0,4 | 0,5 | |
| Methylparaben | | | 0,3 | 0,2 |
| Ethylparaben | | | 0,1 | |
| DMDM Hydantoin | 0,1 | 0,1 | | |
| lodopropynyl Butylcarbamat | | | 0,1 | 0,2 |
| Parfüm | | 0,3 | 0,2 | 0,3 |
| Wasser | ad 100 | ad 100 | ad 100 | ad 100 |

## Patentansprüche

1. Kosmetische Selbstbräunungszubereitung, welche Dihydroxyaceton enthält und **dadurch gekennzeichnet ist, daß** sie ferner ein oder mehrere Ligninsulfonate enthält.

2. Zubereitung nach Anspruch 1, **dadurch gekennzeichnet, daß** sie in Form von O/W-Emulsionen vorliegt.

3. Zubereitung nach Anspruch 2, **dadurch gekennzeichnet, daß** sie eine Tröpfchengröße der inneren Phase von mehr als 500 nm, besonders bevorzugt von mehr als 1000 nm aufweist,

4. Zubereitung nach einem der Ansprüche 2 oder 3, **dadurch gekennzeichnet, daß** sie Cyclomethicone enthält.

5. Zubereitung nach einem der vorhergehenden Ansprüche, **dadurch gekennzeichnet, daß** als Ligninsulfonat Calcium-, Natrium- und/oder Ammoniumligninsulfonat gewählt wird.

6. Zubereitung nach einem der vorhergehenden Ansprüche, **dadurch gekennzeichnet, daß** der Gehalt an einem oder mehreren Ligninsulfonaten aus dem Bereich von 0,01 bis 20 Gew.-% - bezogen auf das Gesamtgewicht der Zubereitung - gewählt wird.

7. Zubereitung nach einem der vorhergehenden Ansprüche, **dadurch gekennzeichnet, daß** der Gehalt an einem oder mehreren Ligninsulfonaten aus dem Bereich von 0,5 bis 15 Gew.-% - bezogen auf das Gesamtgewicht der Zubereitung - gewählt wird.

8. Zubereitung nach einem der vorhergehenden Ansprüche, **dadurch gekennzeichnet, daß** der Gehalt an einem oder mehreren Ligninsulfonaten aus dem Bereich von 1 bis 7 Gew.-% - bezogen auf das Gesamtgewicht der Zubereitung - gewählt wird.

9. Zubereitung nach einem der vorhergehenden Ansprüche, **dadurch gekennzeichnet, daß** sie (bezogen auf das Gesamtgewicht der Zubereitung) weniger als 5 Gew.-% an UV-Filtersubstanzen enthält.

10. Zubereitung nach einem der vorhergehenden Ansprüche, **dadurch gekennzeichnet, daß** der Gehalt an Dihydroxyaceton aus dem Bereich von 0,01 bis 20 Gew.-% - bezogen auf das Gesamtgewicht der Zubereitung - gewählt wird.

11. Zubereitung nach einem der vorhergehenden Ansprüche, **dadurch gekennzeichnet, daß** der Gehalt an Dihydroxyaceton aus dem Bereich von 0,5 bis 15 Gew.-%- bezogen auf das Gesamtgewicht der Zubereitung - gewählt wird.

12. Zubereitung nach einem der vorhergehenden Ansprüche, **dadurch gekennzeichnet, daß** der Gehalt an Dihydroxyaceton aus dem Bereich von 1 bis 10 Gew.-% - bezogen auf das Gesamtgewicht der Zubereitung - gewählt wird.
